Europäisches Patentamt

European Patent Office

Office européen des brevets

(19)

(11) Publication number: **0 023 420**
**B1**

## EUROPEAN PATENT SPECIFICATION

(12)

(45) Date of publication of patent specification: **19.10.83**

(51) Int. Cl.³: **C 07 D 209/48**

(21) Application number: **80302533.7**

(22) Date of filing: **24.07.80**

(54) Process for preparing N,N'-(1,2-ethylene)-bis-tetrabromophthalimide.

(30) Priority: **26.07.79 US 61103**
**26.07.79 US 61160**

(43) Date of publication of application:
**04.02.81 Bulletin 81/5**

(45) Publication of the grant of the patent:
**19.10.83 Bulletin 83/42**

(84) Designated Contracting States:
**BE DE FR GB LU NL**

(56) References cited:
**US - A - 3 624 024**
**US - A - 3 966 726**

**Methoden der organischen Chemie Band V/4,**
**Halogenverbindungen, p. 288**

(73) Proprietor: **ETHYL CORPORATION**
**330 South Fourth Street P.O. Box 2189**
**Richmond Virginia 23219 (US)**

(72) Inventor: **Dotson, Anderson O.**
**Box 216AL**
**Route 3 Monroe Louisiana 71207 (US)**
Inventor: **Lee, Chien Yung**
**36 Beacon Hill Drive**
**East Brunswick New Jersey 08816 (US)**
Inventor: **Wolford, Lionel T.**
**314 Rowland Drive**
**Monroe Louisiana 71207 (US)**
Inventor: **Wadsworth, Francis T.**
**2711 Deborah Drive**
**Monroe Louisiana 71201 (US)**

(74) Representative: **Harrison, David Christopher et al,**
**MEWBURN ELLIS & CO 2/3 Cursitor Street**
**London EC4A 1BQ (GB)**

**The file contains technical information submitted after the application was filed and not included in this specification**

Courier Press, Leamington Spa, England.

# Process for preparing N,N'-(1,2-ethylene)-bis-tetrabromophthalimide

This invention relates to a novel process for preparing N,N'-(1,2-ethylene)-bis-tetrabromophthalimide and more particularly relates to a process for preparing this compound by the bromination of N,N'-(1,2-ethylene)-bis-phthalimide.

As shown in U.S. patents 3,873,567 (Cyba), 4,087,441 (Lee), 4,092,345 (Wolford et al.), 4,125,535 (Wolford), and 4,140,862 (Dobson et al.), British patent 1,287,934 (Raychem), and in Sydney M. Spatz and Herman Stone, "Some N-Substituted Tetrabromophthalimide Fire-Retardant Additives", INDUSTRIAL AND ENGINEERING CHEMISTRY PRODUCT RESEARCH AND DEVELOPMENT, Volume 8, pp. 397—398 (1969), it is known that N,N'-hydrocarbon-bis-tetrabromophthalimides can be prepared by reacting a tetrabromophthalic anhydride, acid, or half-ester with the appropriate diamine. Some of these processes are more economical than others, but all leave something to be desired from the economical standpoint. Another disadvantage of the processes is that most of them lead to the formation of a yellow product, and the few that produce a white product are not commercially attractive enough.

It is also known that reasonably simple aromatic molecules, such as phthalic anhydride, can be brominated by various techniques. A particularly valuable technique involves the use of oleum, as disclosed in U.S. patents 3,382,254 (Jenkner et al.), 3,567,739 (Sanger) and 3,875,186 (Hein et al.), British patent 1,084,375 (Kalk), and German patents 1,039,052 (Guthke) and 1,125,415 (Hahn). However, it has not previously been suggested to apply these known bromination techniques to the bromination of relatively complex aromatic molecules, such as N,N'-hydrocarbon-bis-phthalimides, whether halogenated or not which would be expected to suffer degradation when subjected to bromination in oleum.

An object of this invention is to provide an economical process for preparing N,N'-(1,2-ethylene)-bis-tetrabromophthalimide which involves the bromination of N,N'-(1,2-ethylene)-bis-phthalimide.

In order to achieve this economy, not least because the products are white, we brominate N,N'-(1,2-ethylene)-bis-phthalimide in 40—80% oleum, that is to say, oleum containing 40—80% excess $SO_3$. Relatively mild conditions can be used and, contrary to what would have been expected, the bromination process of the invention results in the preparation of N,N'-(1,2-ethylene)-bis-tetrabromophthalimide instead of degrading the N,N'-(1,2-ethylene)-bis-phthalimide. The process is particularly advantageous in that it is an economical means of preparing the desired product from readily available raw material. When a silver catalyst is employed, the process has the additional advantage of producing lower tetrahydrofuran solubles (i.e., fewer by-products) than comparable processes utilizing different catalysts, and it permits better utilization of the sulfur trioxide and easier clean-up of the brominated product.

The brominating agent used in the process of the invention is usually bromine, although other brominating agents, such as bromine chloride, can be employed if desired. The amount of bromine used should be at least the amount theoretically required to achieve complete bromination of the aromatic rings of the starting material, i.e., an amount that provides one bromine atom for each available aromatic carbon atom. There is no maximum to the amount of bromine that can be used, but there is no advantage to using unnecessarily large amounts of bromine. Therefore, it is preferable to employ amounts of bromine that provide about 1—1.5, most preferably about 1.05—1.15, atoms of bromine per available aromatic carbon atom. It is thus preferable to use about 4—6 mols, most preferably about 4.2—4.6 mols, of bromine per mol of N,N'-(1,2-ethylene)-bis-phthalimide. Excellent results are obtained by using bromine in an amount that is about 10% more than the amount theoretically required for complete bromination.

The oleum used in the practice of the invention is a solution of 40—80% by weight of sulfur trioxide in concentrated sulfuric acid. Because of readier availability as well as effectiveness, a 60—70% oleum is preferred. The oleum is employed in an amount such as to provide at least one molecule of sulfur trioxide per available aromatic carbon atom and is preferably used in larger amounts, since the reaction rate increases with an increased concentration of sulfur trioxide in the reaction mixture. Ordinarily the oleum is utilized in an amount such as to provide 1 to 1.9, preferably 1.25 to 1.75, molecules of sulfur trioxide per available aromatic carbon atom. Thus, it is preferable to use 8 to 15 mols, most preferably 10 to 14 mols, of sulfur trioxide per mole of N,N'-(1,2-ethylene)-bis-phthalimide. Excellent results are obtained by using about 50% more sulfur trioxide than is theoretically required for complete sulfonation of the aromatic rings of the imide. When an oleum containing the desired amount of sulfur trioxide is not readily available, the desired concentration of sulfur trioxide can be attained by using additional sulfur trioxide with an oleum having a low sulfur trioxide content.

In this reaction we find that the bromination can be accomplished under relatively mild conditions, so it is not necessary to use very high temperatures or pressures in the practice of the invention. Ordinarily the desired bromination can be achieved by heating the reactants at 35—60°C., preferably about 55°C., for at least about 3 hours, although it is sometimes desirable to use higher temperatures, e.g., 70—90°C., to complete the bromination when the concentration of sulfur trioxide in the reaction mixture has been relatively low during the initial stage of the reaction. Such relatively low concentrations in the reaction mixture may occur, for example, if a whole charge of N,N'-(1,2-ethylene)-

bis-phthalimide is initially present, as distinct to being added gradually to the oleum.

The reaction may be conducted under atmospheric or superatmospheric pressure, with or without a catalyst. When pressure is employed,it is ordinarily only the slight pressure created by a back pressure device, although higher pressures may be used if desired. When a catalyst is employed, it may be any suitable bromination catalyst, e.g., sodium nitrate, nitrogen dioxide, aluminium, iron, an iron-iodine mixture or a silver catalyst. Catalysts, when employed, are normally used in concentrations of 0.1—2%, based on the weight of the N,N'-1,2-ethylene-bis-phthalimide. Particularly good results are obtained when the catalyst is a 50 : 50 mixture of iron and iodine, and even better results are obtained when the catalyst is a silver catalyst, e.g., silver or any inorganic or organometallic silver compound capable of catalyzing the bromination of aromatic carbons. Because of availability as well as effectiveness, the preferred silver catalysts are silver, silver nitrate, silver sulfate, silver carbonate, silver acetate, silver chloride, silver bromide, and mixtures thereof. Silver nitrate is particularly preferred.

The manner of admixing the reactants is not critical, although the reaction is much more efficient when the reactants are admixed in such a manner as to maximize the concentration of sulfur trioxide at all times. One suitable method of conducting the reaction is to dissolve the N,N'-1,2-ethylene-bis-phthalimide and optional catalyst in the oleum, heat the solution to 35—60°C., add the brominating agent thereto over a period of 3—10 hours, maintain the reaction mixture at 35—60°C. for an additional 0—5 hours, raise the reaction temperature to 70—90°C., and maintain the reaction mixture at about 70—90°C. for about 1—3 hours.

A preferred method of conducting the reaction is to charge optional catalyst and 45—75% of the oleum to a reaction vessel, heat the oleum and optional catalyst to 35—60°C., and simultaneously add the bromine and a solution of the N,N'-1,2-ethylene-bis-phthalimide in the remainder of the oleum over a period of 3—5 hours.

When the bromination has been completed, unreacted materials and by-products are removed by conventional techniques to isolate the N,N' - 1,2 - ethylene - bis - tetrabromophthalimide product. Conveniently the product is isolated by raising the temperature to 100—140°C. to distill of the excess bromine and sulfur trioxide, cooling to room temperature, filtering, washing with water until the product is free of acid, and drying.

The following examples are given to illustrate the invention. Unless otherwise specified, quantities mentioned are quantities by weight.

## Example I

Charge a suitable reaction vessel with 40 parts of N,N'-(1,2-ethylene)-bis-phthalimide, 0.25 parts of iodine, 0.25 parts of iron, and 100 parts of 30% oleum. Stir the mixture, cool it to 150°C. and add 144 parts of sulfur trioxide while stirring which brings the oleum to effectively 65% oleum. Heat the reaction mixture to 50°C. and add 94 parts of bromine over a period of seven hours. After completing addition of the bromine, heat the reaction mixture to 100°C. and maintain this temperature for one hour. Then cool the reaction mixture to 40°C., and set up a Dean Stark trap to distill off sulfur trioxide, and heat the reaction mixture to 120°C, while collecting sulfur trioxide with the Dean Stark trap. Cool the reaction mixture to 25°C., filter, wash the solid product three times with 100 parts of water, and dry overnight at 100°C. in a vacuum oven. The process results in a 91% yield of a white product having a melting point of 458—461°C., a bromine content of 66.05%, and an IR spectrum consistent for N,N'-(1,2-ethylene)-bis-tetrabromophthalimide.

## Example II

Charge 35 parts of N,N'-(1,2-ethylene)-bis-phthalimide and 244 parts of 65% oleum to a suitable reaction vessel equipped with a back pressure system including a check valve. Heat the reaction mixture to 55°C. and add 80.5 parts of bromine over a period of six hours and ten minutes. Maintain the reaction temperature at 55°C. for an additional 40 minutes, heat to 70°C., maintain the temperature at 70°C. for one hour, heat to 100°C., maintain the temperature at 100°C. for one hour, and cool to 80°C. Place a Dean Stark trap in the system and heat to 140°C. while removing excess sulfur trioxide. Then cool the reaction mixture to 20°C., filter, wash the solid product twice with 200 parts of water, and dry at 100°C. in a vacuum oven. The process results in an 88% yield of white N,N'-(1,2-ethylene)-bis-tetrabromophthalimide having tetrahydrofuran solubles content of 0.3% and melting point of 466—472°C.

## Example III

Charge 35 parts of N,N'-(1,2-ethylene)-bis-phthalimide, 0.035 parts of sodium nitrate, and 244 parts of 65% oleum to a suitable reaction vessel equipped with a back pressure system including a check valve. Heat the reaction mixture to 55°C. and add 80.5 parts of bromine over a period of five hours. Maintain the reaction mixture at 55°C. for an additional two hours, heat to 70°C., maintain the temperature at 70°C. for three hours, heat to 100°C. maintain the temperature at 100°C. for one hour, and cool to 80°C. Place a Dean Stark trap into the system and heat to 140°C. while removing excess sulfur trioxide. Then cool the reaction mixture to 20°C., filter, wash the solid product twice with 200 parts of water, and dry at 100°C. in a vacuum oven. The process results in an 85% yield of white N,N'-(1,2-ethylene)-bis-tetrabromophthalimide having a tetrahydrofuran solubles content of 0.3% and a melting point of 466—472°C.

## Example IV

Charge 40 parts of N,N'-(1,2-ethylene)-bis-phthalimide, 0.04 part of iodine, 0.04 part of iron, and 244 parts of 65% oleum to a suitable reaction vessel equipped with a back pressure system including a check valve and cold trap. Heat the reaction mixture to 55°C. and add 92 parts of bromine over a period of six hours. Maintain the reaction temperature at 55°C. for an additional 30 minutes, heat to 70°C., maintain the temperature at 70°C. for three hours, heat to 100°C., maintain the temperature at 100°C. for one hour, and cool to 80°C. Place a Dean Stark trap into the system and heat to 140°C in stages while removing excess bromine and sulfur trioxide. Then cool the reaction mixture to 20°C., filter, wash the solid product twice with 200 parts of water, and dry in a vacuum oven at 100°C. The process results in a 96% yield of white N,N'-(1,2-ethylene)-bis-tetrabromophthalimide having a tetrahydrofuran solubles content of 0.2% and a melting point of 471—476°C.

## Example V

Charge 40 parts of N,N'-(1,2-ethylene)-bis-phthalimide, 0.08 part of silver acetate, and 244 parts of 65% oleum to a suitable reaction vessel equipped with a back pressure system. Heat the reaction mixture to 55°C. and add 92 parts of bromine over a period of six hours. Maintain the reaction temperature at 55°C. for an additional 45 minutes, heat to 70°C., maintain the temeprature at 70°C. for three hours, heat at 97°C. for another hour, and cool to 70°C. Place a Dean Stark trap into the system and heat to 140°C. while removing excess bromine and sulfur trioxide. Then cool the reaction mixture to 20°C., filter, wash the solid product twice with 200 parts of water, and dry. The process results in an 86% yield of white N,N'-(1,2-ethylene)-bis-tetrabromophthalimide having a tetrahydrofuran solubles content of 0.05% and a melting point of 470—472°C.

## Example VI

Charge 40 parts of N,N'-(1,2-ethylene)-bis-phthalimide, 0.04 part of silver nitrate, and 244 parts of 65% oleum to a suitable reaction vessel equipped with a back pressure system. Heat the reaction mixture to 55°C. and add 92 parts of bromine over a period of five hours. Maintain the reaction temperature at 55°C. for an additional 20 minutes, heat to 70°C., maintain at 70°C. for three hours, heat at 100°C. for one hour, and cool to 80°C. Place a Dean Stark trap into the system and heat to 140°C. while removing excess sulfur trioxide. Then cool the reaction mixture to 20°C., filter, wash the solid product twice with 200 parts of water, and dry. The process results in a 95% yield of white N,N-(1,2-ethylene)-bis-tetrabromophthalimide having a tetrahydrofuran solubles content of 0.05% and a melting point of 477—482°C. The process also leads to the recovery of 90.4% of the unreacted sulfur trioxide.

## Example VII

Charge 35 parts of N,N'-(1,2-ethylene)-bis-phthalimide and 244 parts of 65% oleum to a suitable reaction vessel equipped with a back pressure system. Heat the reaction mixture of 55°C. and add 80.5 parts of bromine over a period of six hours and ten minutes. Maintain the reaction temperature at 55°C. for an additoinal 40 minutes, heat to 70°C., maintain the temperature at 70°C. for one hour, heat to 100°C., maintain the temperature at 100°C for one hour, and cool to 80°C. Place a Dean Stark trap in the system and heat to 140°C. while removing excess sulfur trioxide. Then cool the reaction mixture to 20°C., filter, wash the solid product twice with 200 parts of water, and dry. The process results in an 88% yield of whilte N,N'-(1,2-ethylene)-bis-tetrabromophthalimide having tetrahydrofuran solubles content of 0.3% and a melting point of 466—472°C. Only 40% of the unreacted sulfur trioxide is recovered.

## Example VIII

Charge 35 parts of N,N'-(1,2-ethylene)-bis-phthalimide, 0.035 part of sodium nitrate, and 244 parts of 65% oleum to a suitable reaction vessel equipped with a back pressure system. Heat the reaction mixture to 55°C. and add 80.5 parts of bromine over a period of five hours. Maintain the reaction mixture at 55°C. for an additional two hours, heat to 70°C., maintain the temperature at 70°C. for three hours, heat to 100°C., maintain the temperature at 100°C. for one hour, and cool to 80°C. Place a Dean Stark trap into the system and heat to 140°C. while removing excess sulfur trioxide. Then cool the reaction mixture to 20°C., filter, wash the solid product twice with 200 parts of water, and dry. The process results in an 85% yield of white N,N'-(1,2-ethylene)-bis-tetrabromophthalimide having a tetrahydrofuran solubles content of 0.3% and a melting point of 466—472°C. Only 52% of the unreacted sulfur trioxide is recovered.

## Example IX

Charge 40 parts of N,N'-(1,2-ethylene)-bis-phthalimide, 0.04 part of iodine, 0.04 part of iron, and 244 parts of 65% oleum to a suitable reaction vessel equipped with a back pressure system. Heat the reaction mixture to 55°C. and add 92 parts of bromine over a period of six hours. Maintain the reaction temperature at 55°C. for an additional 30 minutes, heat to 70°C., maintain the temperature at 70°C. for three hours, heat to 100°C., maintain the temperature at 100°C. for one hour, and cool to 80°C. Place a Dean Stark trap into the system and heat to 140°C. while removing excess bromine and sulfur trioxide. Then cool the reaction mixture to 20°C., filter, wash the solid product twice with 200 parts of water, and dry. The process results in a 96% yield of white N,N'-(1,2-ethylene)-bis-tetrabromophthalimide having a tetrahydrofuran solubles content of 0.2% and a melting point of 471—476°C. The process also leads to the recovery of 90% of the unreacted sulfur trioxide.

## Claims

1. A process for preparing N,N'-(1,2-ethylene)-bis-tetrabromophthalimide characterised in brominating N,N'-(1,2-ethylene)-bis-phthalimide in oleum containing 40—80% excess $SO_3$.

2. A process according to claim 1, wherein oleum containing 60—70% excess $SO_3$ is used.

3. A process according to claim 1 or claim 2 wherein the reaction mixture contains 1 to 1.5 atoms of bromine and 1 to 1.9 molecules of sulfur trioxide per available aromatic carbon atom in the bis-phthalimide.

4. A process according to claim 3, wherein the reaction mixture contains 1.05 to 1.15 atoms of bromine and 1.25 to 1.75 molecules of sulfur trioxide per available aromatic carbon atom in the bis-phthalimide.

5. A process according to claim 1 or claim2 wherein 4—6 molar proportions of bromine are reacted with one molar proportion of the bis-phthalimide in an amount of the oleum containing 8—15 molar proportions of sulfur trioxide by heating 45—75% of the oleum to a temperature of 35—60°C., then simultaneously adding thereto over a period of 3—5 hours the bromine and a solution of the bis-phthalimide in the remainder of the oleum, and removing unreacted materials and by-products to isolate the resultant N,N'-(1,2-ethylene)-bis-tetrabromophthalimide.

6. A process according to any one of the preceding claims, wherein the bromination is conducted in the presence of a silver or an iron/iodine catalyst.

7. A process according to claim 6, wherein the catalyst is silver nitrate.

## Revendications

1. Un procédé pour la préparation du N,N'-(ethylène-1,2)-bis-tetrabromophtalimide, caractérisé par la bromation du N,N'-(ethylène-1,2)-bis-phtalimide dans un oléum contenant un excès de $SO_3$ de 40 à 80%.

2. Un procédé selon la revendication 1 dans lequel on emploie un oléum contenant un excès de $SO_3$ de 60 à 70%.

3. Un procédè selon l'une des revendications 1 ou 2, dans lequel le mélange réactionnel contient 1 à 1,5 atome de brome et 1 à 1,9 molécule de trioxyde de soufre par atome de carbone aromatique disponible dans le bis-phtalimide.

4. Un procédé selon la revendication 3, dans lequel le mélange réactionnel contient 1,05 à 1,15 atome de brome et 1,25 à 1,75 molécule de trioxyde de soufre par atome de carbone aromatique disponible dans le bis-phtalimide.

5. Un procédé selon l'une des revendications 1 ou 2, dans lequel on fait réagir 4 à 6 proportions molaires de brome avec une proportion molaire du bis-phtalimide dans une quantité d'oléum contenant 8 à 15 proportions molaires de trioxyde de soufre par chauffage de l'oléum à 45—75% à une température de 35—60°C, puis on ajoute simultanément en une période de 3 à 5 h du brome, et d'une solution du bis-phtalimide dans le reste de l'oléum et on élimine les matières n'ayant pas réagi et les sous-produits pour isoler le N,N'-(ethylène-1,2)-bistetrabromophtalimide obtenu.

6. Un procédé selon lequel l'une quelconque des revendications précédentes, dans lequel la bromation est effectuée en présence d'un catalyseur à l'argent et/ou au fer/iode.

7. Un procédé selon la revendication 6 dans lequel le catalyseur est le nitrate d'argent.

## Patentansprüche

1. Verfahren zur Herstellung von N,N'-(1,2-Ethylen)-tetrabromphthalimid, gekennzeichnet durch Bromieren von N,N'-(1,2-Ethylen)-bis-phthalimid in Oleum mit 40—80% überschüssigem $SO_3$.

2. Verfahren nach Anspruch 1, bei dem Oleum mit 60—70%igem Überschuß an $SO_3$ verwendet wird.

3. Verfahren nach Anspruch 1 oder Anspruch 2, bei dem das Reaktionsgemisch 1 bis 1,5 Atome Brom und 1 bis 1,9 Moleküle Schwefeltrioxid pro vefügbarem aromatischen Kohlenstoffatom im Bisphthalimid enthält.

4. Verfahren nach Anspruch 3, bei dem das Reaktionsgemisch 1,05 bis 1,15 Atome Brom und 1,25 bis 1,75 Moleküle Schwefeltrioxid pro verfügbarem aromatischem Kohlenstoffatom im Bisphthalimid enthält.

5. Verfahren nach Anspruch 1 oder Anspruch 2, bei dem 4 bis 6 Mol-Anteile Brom mit einem Mol-Anteil des Bis-phthalimids in einer Menge des Oleums, die 8 bis 15 Mol-Anteile Schwefeltrioxid enthält, durch Erwärmen von 45 bis 75% des Oleums auf eine Temperatur von 35 bis 60°C umgesetzt werden, dann glkeichzeitig hierzu über einen Zeitraum von 3 bis 5 h das Brom und eine Lösung des Bis-phthalimids im Rest des Oleums zugesetzt und nicht umgesetzte Materialien und Nebenprodukte entfernt werden, um das anfallende N,N'-(1,2-Ethylen)-bis-tetrabromphthalimid zu isolieren.

6. Verfahren nach irgend einem der vorhergehenden Ansprüche, bei dem die Bromierung in Gegenwart eines Silber- oder eines Eisen/Jod-Katalysators durchgeführt wird.

7. Verfahren nach Anspruch 6, bei dem der Katalysator Silbernitrat ist.